# EUROPÄISCHE PATENTSCHRIFT

(11) **EP 0 383 234 B1**
(45) Veröffentlichungstag und Bekanntmachung des Hinweises auf die Patenterteilung: **30.11.1994**
(21) Anmeldenummer: 90102716.9
(22) Anmeldetag: 12.02.1990
(51) Int. Cl.: C07K 3/22, A61L 2/04, C07K 15/06, A61K 37/02

(54) **Pasteurisiertes, gereinigtes von Willebrand-Faktor-Konzentrat und Verfahren zu seiner Herstellung**
Pasteurised purified von Willebrand factor concentrate, and method for its preparation
Concentré pasteurisé, purifié du facteur Von Willebrand et son procédé de préparation

(30) Priorität: 14.02.1989 DE 3904354
(43) Veröffentlichungstag der Anmeldung: 22.08.1990
(73) Patentinhaber: BEHRINGWERKE Aktiengesellschaft, 35001 Marburg (DE)
(72) Erfinder: Heimburger, Norbert, Prof. Dr., D-3550 Marburg (DE); Kumpe, Gerhard, D-3552 Wetter (DE); Wellner, Klaus, D-3550 Marburg (DE)

(56) Entgegenhaltungen:
- EP-A- 0 106 269
- US-A- 4 774 323

## Beschreibung

Die Erfindung betrifft ein Verfahren zur Herstellung eines gereinigten und pasteurisierten von Willebrand-Faktor-Konzentrats sowie ein nach diesem Verfahren hergestelltes solches Konzentrat, das für die Behandlung des von Willebrand-Syndroms geeignet ist.

Dieses Krankheitsbild ist durch einen kongenitalen Mangel und/oder Defekt des von Willebrand-Proteins charakterisiert.

Nach einem reinen und virussicheren von Willebrand-Faktor-Konzentrat besteht ein Bedarf, da zur Behandlung der Hämophilie A immer besser gereinigte Faktor VIII:C-Konzentrate zur Anwendung gelangen, die den von Willebrand-Faktor nur noch in Spuren enthalten.

Da die von Willebrand-Patienten lebenslang und zum Teil mit hohen Dosen therapiert werden müssen, ist eine hohe Reinheit und Sicherheit des Präparates indiziert. Vorteilhaft sind Präparationen, die arm an Fibrinogen, Immunglobulinen und Isoagglutininen sind.

Im Plasma zirkuliert der von Willebrand-Faktor in einer Konzentration von 5 - 10 mg/l und in Form eines nicht kovalent gebundenen Komplexes mit dem Faktor VIII, dem sogenannten antihämophilen Globulin. Im Kryopräzipitat ist der von Willebrand-Faktor als von Willebrand-Faktor/ Faktor VIII-Komplex stark angereichert und kann daraus oder aus Plasma oder Plasmafraktionen mit bekannten Fraktionierungsmethoden isoliert werden.

Aus der deutschen Offenlegungsschrift 35 04 385 (USP 4,578,218) ist ein Verfahren zur Behandlung von Faktor VIII-Komplex bekannt, worin eine Faktor VIII-Präparation an eine unlösliche Matrix gebunden wird, die freie Sulfatgruppen trägt, beispielsweise Dextransulfat, wobei jedoch offenbar keine Trennung des Faktor VIII-Komplexes in Faktor VIII:C und von Willebrand-Faktor möglich ist.

In GB 2 079 292 ist ein Verfahren zur Gewinnung eines von Willebrand-Faktors aus Kryopräzipitat beschrieben, das jedoch ebenfalls nicht Faktor VIII:C von dem von Willebrand-Faktor trennt.

In der EP 0 022 052 (USP 4,210,580) wird ein Verfahren beschrieben, worin Plasma mit Natrium-Heparin behandelt wird, worauf Fibronectin zusammen mit von Willebrand-Faktor ausfällt. Aus dem Überstand wird antihämophiler Faktor gewonnen. Das Präzipitat wird an DEAE-Cellulose chromatographiert und Fibronectin gewonnen. Es ist angegeben, daß, wenn zur Chromatographie Agarosegel verwendet wird, von Willebrand-Faktor im "void volume" eluiert wird. Heparin jedoch ist in den verwendeten Mengen kostspielig und eine Gelfiltration ein Engpaß für eine Herstellung im Produktionsmaßstab. Außerdem wird mit dem toxischen KSCN gearbeitet.

In der europäischen Patentschrift 0 083 483 ist zum Stand der Technik angegeben, daß aus J.Lab.Clin.Med. 93, 40 (1979) ein Verfahren zur Trennung von Faktor VIII:C und von Willebrand-Faktor beschrieben sei, wobei die Trennung durch Immunadsorption bewirkt wird. In diesem Verfahren wird jedoch lediglich der Faktor VIII:C in genügender Reinheit erhalten.

Ein weiteres Verfahren zur Trennung dieser beiden Faktoren ist in Brit.J.Hematol. 43, 669 (1979) beschrieben, wobei Aminohexyl-Agarose verwendet wird. Auch dieses Verfahren ist ungeeignet zur Gewinnung von von Willebrand-Faktor.

In der EP 0 083 483 selbst wird ein Verfahren zur Gewinnung von Faktor VIII:C beschrieben, der nur geringe Mengen an von Willebrand-Faktor enthält. Dort wird jedoch kein Verfahren zur Gewinnung des von Willebrand-Faktors beschrieben.

Allen diesen Verfahren ist gemeinsam, daß sie nicht zu einer vollständigen Dissoziation des Komplexes mit nachfolgender quantitativen Freisetzung eines nativen F VIII:C und vWF führen. Keines dieser Verfahren beschreibt ein pasteurisiertes und damit virussicheres Produkt. Um den vWF während der Reinigung vor proteolytischer Zersetzung zu schützen, werden in diesen Verfahren toxische Substanzen wie DFP und Sojabohnen-Trypsininhibitor oder auch Puffer wie KSCN und NaN₃ verwendet. Schließlich haben diese Verfahren Nachteile, weil sie Schritte enthalten, die einen Engpaß für die Produktion im Großen darstellen. Dazu gehören beispielsweise Gelfiltrationen, d. h. Trennung nach dem Molekulargewicht oder chromatographische Schritte unter Verwendung eines Salzgradienten für die Elution.

Die vorliegende Erfindung beschreibt ein Verfahren, mit dem es überraschenderweise gelingt, den Komplex aus Faktor VIII:C und von Willebrand-Faktor zu dissoziieren und den von Willebrand-Faktor gereinigt und pasteurisiert mit hoher Ausbeute zu isolieren. Ziel dieser Erfindung ist die Gewinnung eines gereinigten, pasteurisierten und damit virussicheren Gerinnungstherapeutikums zur Behandlung des Von Willebrand-Syndroms.

Gegenstand der Erfindung ist ein Verfahren zur Herstellung eines pasteurisierten von Willebrand-Faktor-Konzentrates, dadurch gekennzeichnet daß eine Lösung, die den von Willebrand-Faktor (vWF) als Komplex mit dem F VIII:C in einem calcium- und aminosäurehaltigen Puffer von pH 5,5 bis 6,5 und einer. Kohlenhydratkonzentration von 5 - 30 % w/w enthält, mit einem Anionenaustauscher behandelt wird, an den sich der F VIII:C bindet, und daß aus der Lösung das von Willebrand-Faktor-Konzentrat gewonnen wird.

Als Ausgangsmaterial für die Herstellung eines vWF-Konzentrates können Lösungen verwendet werden, in denen der vWF als Komplex mit dem F VIII:C vorliegt, z. B. Plasma und daraus gewonnene Fraktionen wie Kryopräzipitat, Cohn-Fraktion I oder auch Überstände und Extrakte von Zellkulturen.

Das Ausgangsmaterial, vorzugsweise Kryopräzipitat oder eine daraus gewonnene Zwischenfraktion, kann pasteurisiert worden sein.

Der vWF kann gegen eine thermische Inaktivierung während der Pasteurisierung durch die Zusätze von Kohlenhydraten, vorzugsweise Saccharose, vorzugsweise in Konzentrationen von 10 - 60 % (w/w), und/oder Aminosäuren, vorzugsweise Glycin, vorzugsweise in den Konzentrationen von 0,5 - 3,0 mol/l, und/oder Calciumsalzen, vorzugsweise 1 - 20 mmol/l, geschützt werden. Durch diese Maßnahmen kann gleichzeitig auch die Ausfällung säureempfindlicher Proteine z. B. des Fibronectins verhindert werden.

Die Kohlenhydrate dienen nicht nur als Schutz der Proteine vor einer thermischen Inaktivierung bzw. Denaturierung, sondern auch als Lösungsvermittler im sauren pH-Bereich von 6,5 bis 5,5, und hier im besonderen für das Fibrinogen und den vWF, indem sie überraschenderweise eine Fällung verhindern.

Man kann den vWF nach Adsorption des F VIII:C an den Ionenaustauscher bei pH 5,5 in Lösung halten, daraus das Fibrinogen durch Zugabe von Glycin in Konzentrationen von 0,5 - 3 mol/l, vorzugsweise 2,7 mol/l, abtrennen und aus dem Glycinüberstand den vWF mit NaCl-Konzentrationen entsprechend 2 - 15 % (w/v), vorzugsweise 6 % (w/v), präzipitieren.

Der vWF kann als vorgereinigtes Zwischenprodukt zur Erhöhung der Virussicherheit ein zweitesmal pasteurisiert werden.

Der pasteurisierte und hochgereinigte vWF kann beispielsweise mit Glycin (2 % w/v), Albumin (0,5 %) in Citrat (0,02 mol/l)-NaCl (0,06 mol/l) als Stabilisatoren sterilfiltriert und lyophilisiert werden.

Die Bedingungen zur Dissoziation und Reindarstellung sind auf große Produktionsmaßstäbe übertragbar.

Das erfindungsgemäße Präparat ist im Gegensatz zu antihämophilem Kryopräzipitat, rohem Kryopräzipitat oder Kryofraktionen, die bislang zur Behandlung des vW-Syndroms zur Anwendung kamen, nahezu frei von Ballastproteinen.

Den geforderten hohen Anforderungen bezüglich Reinheit, Ausbeute und Virussicherheit genügt das erfindungsgemäße Verfahren: Zusammen mit den Ballastproteinen werden durch den Reinigungsprozeß auch möglicherweise vorhandene Viren eliminiert und über eine Pasteurisierung inaktiviert. Die spezifische Aktivität eines nach dem beschriebenen Verfahren hergestellten Produkts liegt über 100 E F VIIIR:Co F/mg Protein.

Es kann folgendermaßen gearbeitet werden:
Gelöstes Kryopräzipitat, stark angereichert mit dem von Willebrand-Faktor und Faktor VIII, das durch eine AL(OH)₃Adsorption von den Faktoren des Prothrombinkomplexes befreit ist, wird in an sich bekannter Weise durch Zusatz von Kohlenhydraten, Aminosäuren und Calcium-Ionen zum Schutze gegen thermische Inaktivierung stabilisiert und in wässriger Lösung erhitzt, vorzugsweise 10 Std. bei 60°C.

Die pasteurisierte Lösung kann mit einem Puffer physiologischer Leitfähigkeit (12-15 mS) und einem pH-Wert von 5,5 der Zusammensetzung 0,2 mol/l Lysin und 0,2 mol/l Na-Acetat auf das doppelte Volumen verdünnt, der pH-Wert der Lösung auf 5,5 eingestellt und mit einem Anionenaustauscher bei 20°C versetzt werden.

Bei diesen Bedingungen bindet sich der Faktor VIII an basische Ionenaustauscher mit DEAE und QAE als funktioneller Gruppe gebunden an ^{R}Sephadex, ^{R}Sepharose, Cellulose oder ^{R}Fractogel als Matrix, während der vWF in Lösung bleibt. Zu diesem Zweck werden diese Austauscher zuvor mit folgendem Puffer äquilibriert: 0,1 mol/l Na-Acetat, 0,1 mol/l Lysin, 0,017 mol/l NaCl, pH 5,5.

Da unter den genannten Bedingungen der vWF zusammen mit Fibrinogen und Fibronectin im Überstand verbleibt, wird offenbar unter den genannten Bedingungen der Komplex des vWF mit Faktor VIII dissoziiert.

Der mit F VIII beladene Anionenaustauscher kann mit Pufferlösung, enthaltend 0,1 mol/l Lysin, 0,1 mol/l Na-Acetat, 0,017 mol/l NaCl, pH 5,5, oder anderen Puffern mit einer Leitfähigkeit 12 - 15 mS, gewaschen werden. Zur Elution werden Puffer mit hoher Salzkonzentration, z. B. 0,3 - 1 mol/l NaCl oder andere Alkali-, oder Erdalkalihalogenide verwendet.

Das Eluat kann gegebenenfalls zu einem pasteurisierten und hochgereinigten F VIII:C-Konzentrat aufgearbeitet werden.

Die beschriebene Dissoziation und selektive Adsorption im sauren pH-Bereich ist nur dann möglich, wenn das Fibrinogen, das den Hauptanteil des Proteins in der Lösung ausmacht, nicht ausfällt; denn bekanntlich können Euglobuline, zu denen das Fibrinogen auch zählt, in wässriger Lösung durch Ansäuern auf pH 5 bis 5,5 ausgefällt werden.

Das gilt für das erfindungsgemäße Verfahren nicht, weil die von der Pasteurisierung her in der Lösung verbleibenden Kohlenhydrate und das Calcium das Fibrinogen auch bei einem saurem pH in Lösung halten.

Dieser Verfahrensschritt ist ebenfalls Gegenstand und Bestandteil der vorliegenden Erfindung, auch wenn die Pasteurisierung der Proteine zu einem späteren Zeitpunkt erfolgt.

Der von Willebrand-Faktor, frei von Faktor VIII:C- Aktivität, bleibt zusammen mit dem Fibrinogen und dem Fibronectin im Überstand (Batch-Verfahren) bzw. passiert die Säule und kann anschließend durch geeignete Fraktionierungsschritte von den mengenmäßig im großen Überschuß vorhandenen Begleitproteinen abgetrennt werden, beispielsweise durch eine Glycinfraktionierung und NaCl-Präzipitation des Anionenaustauscherüberstandes.

Zu diesem Zweck wird der DEAE-Überstand auf pH 7,3 gestellt und bei 37°C mit 0,5 - 3 mol/l Glycin gefällt, vorzugsweise mit 2,7 mol/l, das Fibrinogen präzipitiert und abgetrennt.

Durch Zusatz von festem oder gelöstem NaCl zum Glycinüberstand bis auf eine Endkonzentration von 2 - 15 % w/v, vorzugsweise 6 % w/v, wird der von Willebrand-Faktor selektiv ausgefällt und beispielsweise in einer Zentrifuge abgetrennt.

Das gelöste Präzipitat kann durch Behandlung mit ^{R}Aerosil noch höher gereinigt werden. Bei einer bestimmten Eiweißkonzentration, die über die OD bei 280 nm eingestellt wird, werden vom Aerosil bevorzugt Begleitproteine des vWF gebunden, nämlich Fibrinogen, Fibronektin und Immunglobuline; auch die unerwünschten Isoagglutinine werden deutlich vermindert. Die Aerosilbehandlung kann sowohl vor als auch nach der Pasteurisierung erfolgen.

Das so hergestellte von Willebrand-Faktor-Präzipitat kann gelöst, mit Saccharose-Glycin versetzt und zur Erhöhung der Virussicherheit erneut 10 Std. auf 60°C erhitzt werden. Diese Pasteurisierung ist überraschenderweise praktisch ohne Aktivitätsverlust möglich. Aus der erkalteten und verdünnten Lösung wird das noch vorhandene Rest-Fibrinogen durch eine Glycinfällung von 0,5 - 3,0 mol/l, vorzugsweise 2,2 mol/l, abgeschieden. Über eine anschließende Kochsalzfällung des Überstandes mit 2 - 15 % w/v, vorzugsweise 8 % w/v, kann der von Willebrand-Faktor in hochreiner Form und auch niedrigem Isoagglutinin-Titer isoliert werden.

Nach Auflösen des von Willebrand-Faktors in einen Puffer aus 0,02 mol/l Citrat, 0,06 mol/l NaCl, pH 6,8 und Stabilisieren durch Zusatz von Aminosäuren und Albumin schließt sich eine Dialyse an; dann wird das Produkt sterilfiltriert und gegebenenfalls lyophilisiert. Die Anreicherung des vWF wurde über die F VIIIR:CoF-Aktivität nach der Agglutinationsmethode bestimmt.

Stabilisierte Plättchen werden in Anwesenheit des F VIIIR:CoF und dem Antibiotikum Ristocetin A agglutiniert.

Durchführung der Bestimmung:
50 µl von Willebrand-Reagenz, Behringwerke (in 1 ml aqua dest. resuspendiert) und 50 µl Plasma bzw. Plasmaverdünnung werden auf einer Glasplatte gemischt und eine Minute bei Raumtemperatur geschwenkt, entweder auf einem Schütteltisch oder per Hand; gute Durchmischung der Probe ist zu beachten. Nach einer Minute Ruhe wird der Agglutinationsgrad mit einer Kochsalzkontrolle verglichen. Die Verdünnungsstufe, die noch positiv gegenüber der Kochsalzkontrolle ist, wird abgelesen und mit der Empfindlichkeit des Reagenzes multipliziert. Man erhält den F VIIIR:CoF-Gehalt in Prozent.

Nachfolgend wird an Beispielen die Herstellung eines pasteurisierten, hochgereinigten von Willebrand Faktor-Konzentrats beschrieben.

### Beispiel 1

### 1. Ausgangsmaterial

1 kg rohes Kryopräzipitat wurde unter Erwärmen auf 30 - 37°C mit 3 l eines Puffers gelöst, der 0,08 mol/l NaCl, 0,27 mol/l Glycin, 0,13 E/ml Antithrombin III und 0,66 USP E/ml Heparin enthielt. Es resultierten 4 l Lösung mit einem pH-Wert von 6,8 - 6,9 und folgenden Zusätzen in folgenden Konzentrationen

| | |
|---|---|
| NaCl | 0,06 mol/l |
| Glycin | 0,2 mol/l |
| AT III | 0,1 E/ml |
| Heparin | 0,5 E/ml. |

### 2. Al(OH)₃-Adsorption

Zu 1000 ml Lösung aus 1 wurden 80 ml einer 1%igen Al(OH)₃Suspension (Behringwerke, Marburg) gegeben und 15 min gerührt, Temperatur: 28 - 30°C. Danach wurde 15 min bei 3000 x g zentrifugiert, der Rückstand verworfen und der Abguß mit Stabilisatoren versetzt und pasteurisiert.

### 3. Stabilisierung und Pasteurisierung

1000 ml Abguß aus 2 wurden wie folgt mit Stabilisatoren versetzt:
5 ml CaCl₂-Lösung, 1 mol/l (5 mmol/l)
1000 g Saccharose (500 g/kg Lösung)
150 g Glycin (2 mol zu 1 l Lösung).

Der pH-Wert wurde mit 2 N NaOH auf 7,3 eingestellt. Durch die Zusätze vergrößerte sich das Volumen der Lösung. Ausgehend von 1 kg Kryopräzipitat wurden 6,8 l stabilisierte Lösung erhalten, die 10 Std. bei 60°C im Wasserbad erhitzt wurde.

### 4. Ionenaustauscher-Behandlung

6,8 l Lösung aus 3 wurden mit 6,8 l eines Puffers, der 0,2 mol/l Na-Acetat, pH 5,5 und 0,2 mol/l Lysin enthielt, verdünnt. Der pH-Wert wurde mit verdünnter Essigsäure auf pH 5,5 eingestellt.

Die Lösung wurde mit 300 ml DEAE-^{R}Sepharose CL 6B versetzt, die mit einem Puffer von pH 5,5, der 0,1 mol/l Na-Acetat, 0,1 mol/l Lysin, 1 g/l NaCl enthielt, äquilibriert worden war. Die Suspension wurde 2 - 3 Stunden bei Raumtemperatur gerührt und der Adsorptionsverlauf über kontinuierliche F VIII-Bestimmungen überwacht. Dann wurde das beladene Harz über einen Nylonfilterbeutel gegeben und abgetrennt, gewaschen, eluiert und das Eluat zu F VIII:C-Konzentrat aufgearbeitet.

### Gewinnung des von Willebrand-Faktors aus dem DEAE-Überstand.

### 5. 2,7 M Glycinfällung

Zur Fällung wurde der DEAE-Abguß mit 2 M NaOH auf pH 6,8 eingestellt, auf 37°C erwärmt und durch Zugabe von 2,1 mol/l kristallinem Glycin (157,5 g) auf eine Endkonzentration von 2,7 mol/l gebracht, da der DEAE-Abguß bereits 0,6 mol/l Glycin aus der pasteurisierten Lösung enthält. Das Glycin wird über 30 min unter Rühren langsam zudosiert. Dabei fällt das Fibrinogen aus und wird abgeschieden. Der Fällungsansatz kühlt sich dabei auf 20 - 25°C ab. Das Fibrinogenpräzipitat wurde durch Zentrifugieren bei 3000 - 5000 x g abgetrennt.

### 6. 6 % NaCl-Fällung

Der von Willebrand-Faktor wurde durch Zugabe von kristallinem NaCl zum 2,7 molaren Glycin-Abguß (60 g/l) gefällt. Die Fällung erfolgte bei Raumtemperatur; das NaCl wurde über 30 min zudosiert und 30 min nachgerührt. Der feine Niederschlag wurde in einer Durchlaufzentrifuge bei 15 000 x g und 10°C und einem Durchsatz von 40 l/Std. abgetrennt.

### 7. Lösen des 6 % NaCl-Rückstandes, Stabilisieren und Pasteurisieren

Der 6 % NaCl-Rückstand wurde mit 60 ml aqua dest. gelöst. Es wurden 82,5 ml einer Lösung mit einer optischen Dichte von 40 bei 280 erhalten. Bei einer optischen Dichte größer ab 50 wird auf diesen Wert verdünnt.

Zur Stabilisierung wurden zu 82,5 ml Lösung 82,5 g Saccharose (1 g/ml) und 12,3 g Glycin (2 mol/l) zugesetzt. Das Volumen der stabilisierten Lösung betrug 140 ml, der pH-Wert wurde mit 2 m NaOH auf 6,8 eingestellt und die stabilisierte Lösung 10 Std. auf 60°C erhitzt.

### 8. Isolierung des pasteurisierten von Willebrand-Faktors aus der Stabilisatorlösung

Verdünnen:
Nach dem Erkalten auf 40°C wurde die pasteurisierte Lösung im Verhältnis 1:3 mit 280 ml eines Puffers verdünnt, der 0,03 mol/l NaCl und 0,02 mol/l tri-Na-Citrat enthielt. Die optische Dichte der Lösung bei 280 nm betrug 7,81 nach Verdünnen.

### Glycinvorfällung (2,2 mol/l)

420 ml der verdünnten und pasteurisierten Lösung, die bereits 0,4 mol/l Glycin enthielt, wurden bei 35°C mit 56,7 g Glycin (1,8 mol/l) versetzt. Die Lösung kühlte während der Fällung (30 min) und der anschließenden Rührzeit (30 min) auf 25°C ab.

Der Niederschlag wurde bei 3000 g abzentrifugiert und verworfen.

### 8 % NaCl-Fällung

Der 2,2 molare Glycin-Überstand (420 ml) wurde mit dem 0,38fachen Volumen eines Fällungsmediums (159,6 ml) das 1,8 mol Glycin und 300 g NaCl im Liter enthielt, versetzt. Die Temperatur bei der Fällung betrug 20°C, die Fällungs- und Rührzeit insgesamt 1 Stunde. Der 8 % NaCl-Rückstand, der den von Willebrand-Faktor HS enthielt, wurde bei 5000 g abzentrifugiert.

Lösen des 8 % NaCl-Rückstandes, Dialyse, Ultrazentrifugation:
Der 8 % NaCl-Rückstand wurde mit 33 ml eines Lösepuffers von pH 6,9 folgender Zusammensetzung gelöst:
- Lösepuffer:: 0,06 mol/l NaCl
0,02 mol/l tri-Na-Citrat
2 % Glycin
0,5 % Human-Albumin.

Die Lösung wurde 2 x 1,5 Std. lang bei 20°C gegen 1,2 l eines Dialyspuffers unter Rühren dialysiert:
- Dialysepuffer:: 0,06 mol/l NaCl
0,02 mol/l tri-Na-Citrat
2 % Glycin
(pH 6,8 - 6,9).

Das Dialysat (69 ml) wurde mit Humanalbumin auf eine Endkonzentration von 0,5 % gebracht und 60 min bei 20°C und 15 000 g klärzentrifugiert.

Zur Sterilfiltration wurde die ultrazentrifugierte Lösung mit Lösepuffer auf 110 ml aufgefüllt.

Sterilfiltration, Konzentrationseinstellung, Abfüllung und Lyophilisation:
Die ultrazentrifugierte Lösung des von Willebrand-Faktor HS-Konzentrates (110 ml) wurde nach Erwärmen auf 30 - 35°C über ein Plattenfilter der Porenweiten 0,45 nm und 0,2 nm sterilfiltriert. Die Lösung enthielt 160 E/ml F VIIIR:CoF-Aktivität.

### Beispiel 2

Gemäß Beispiel 1, Paragraph 6, wurde eine 6 % NaCl-Fällung gewonnen und der von Willebrand-Faktor durch Behandlung mit Aerosil höher gereinigt. Dazu wurde das Präzipitat zunächst in 60 ml aqua dest. gelöst, die optische Dichte (OD) bei 280 nm gemessen und die Lösung danach auf 200 ml mit einer OD von 10 verdünnt. Zu dieser Lösung wurde feuchtes Aerosil 200 gegeben (bezogen auf Trockensubstanz 5 mg/ml) und die Suspension 30 min bei 20°C gerührt; dann wurde das mit Begleitproteinen des von Willebrand-Faktors beladene Aerosil abzentrifugiert und der Überstand, wie in Beispiel 1 ausführlich beschrieben, bis zum Endprodukt aufgearbeitet. Dieses hatte bei unverändert guter Ausbeute eine um den Faktor 2 bis 4 höhere spezifische Aktivität.

## Patentansprüche

1. Verfahren zur Herstellung eines pasteurisierten von Willebrand-Faktor-Konzentrates, dadurch gekennzeichnet, daß eine Lösung, die den von Willebrand-Faktor (vWF) als Komplex mit dem F VIII:C in einem calcium- und aminosäurehaltigen Puffer von pH 5,5 bis 6,5 und einer Kohlenhydratkonzentration von 5 - 30 % w/w enthält, mit einem Anionenaustauscher behandelt wird, an den sich der F VIII:C bindet, und daß aus der Lösung das von Willebrand-Faktor-Konzentrat gewonnen wird.

2. Verfahren nach Anspruch 1, dadurch gekennzeichnet, daß als Lösung, die den von Willebrand-Faktor als Komplex mit dem F VIII:C enthält, Plasma oder eine daraus gewonnene Fraktion, vorzugsweise Kryopräzipitat oder Cohn-Fraktion I, oder ein Überstand oder Extrakt einer Zellkultur verwendet wird.

3. Verfahren nach Anspruch 1, dadurch gekennzeichnet, daß die Lösung, die den von Willebrand-Faktor enthält, pasteurisiert wird.

4. Verfahren nach Anspruch 1, dadurch gekennzeichnet, daß die Lösung des von Willebrand-Faktors pasteurisiert wird und während der Pasteurisierung gegen eine thermische Inaktivierung durch den Zusatz von Saccharose, vorzugsweise in einer Konzentration von 10 - 60 % w/v, Glycin, vorzugsweise in einer Konzentration von 0,5 - 3,0 mol/l, und einem Calciumsalz, vorzugsweise 1-20 mmol/l, geschützt wird und durch solche Zusätze gleichzeitig die Ausfällung säureempfindlicher Proteine, besonders des Fibrogens und Fibronectins, verhindert wird.

5. Verfahren nach einem der Ansprüche 1 bis 4, dadurch gekennzeichnet, daß die von Willebrand-Faktor-Präzipitate, wie sie vor oder nach der Pasteurisierung anfallen, gelöst und mit ^{R}Aerosil behandelt werden.

6. Verfahren nach Anspruch 1, dadurch gekennzeichnet, daß man nach der Ionenaustauscher-Behandlung aus der Lösung Fibrinogen durch Zugabe von Glycin in einer Konzentration von 0,5 bis 3 mol/l, vorzugsweise 2,7 mol/l ausfällt und aus dem Überstand den vWF mit einer NaCl-Konzentration von 2-15 % (w/v), vorzugsweise 6 % (w/v), präzipitiert.

7. Verfahren nach Anspruch 1, dadurch gekennzeichnet, daß der pasteurisierte und gereinigte vWF unter Zusatz von Glycin (2 % w/v), Albumin (0,5 %) in Citrat (0,02 mol/ l)-NaCl (0,06 mol/l) sterilfiltriert und lyophilisiert wird.

## Claims

1. A process for the preparation of a pasteurized von Willebrand factor concentrate, which comprises a solution which contains von Willebrand factor (vWF) as complex with F VIII:C in a buffer of pH 5.5 to 6.5, which contains calcium and amino acids and has a carbohydrate concentration of 5 - 30% w/w, being treated with an anion exchanger to which F VIII:C binds, and the von Willebrand factor concentrate being obtained from the solution.

2. The process as claimed in claim 1, wherein plasma or a fraction obtained therefrom, preferably cryoprecipitate or Cohn fraction I, or a supernatant or extract of a cell culture, is used as solution which contains von Willebrand factor as complex with F VIII:C.

3. The process as claimed in claim 1, wherein the solution which contains von Willebrand factor is pasteurized.

4. The process as claimed in claim 1, wherein the solution of von Willebrand factor is pasteurized and protected from thermal inactivation during the pasteurization by the addition of sucrose, preferably in a concentration of 10-60% w/v, glycine, preferably in a concentration of 0.5-3.0 mol/l, and a calcium salt, preferably 1-20 mmol/l, and the precipitation of acid-sensitive proteins, especially fibrinogen and fibronectin, is simultaneously prevented by such additives.

5. The process as claimed in any one of claims 1 to 4, wherein the von Willebrand factor precipitates which are produced before or after the pasteurization are dissolved and treated with ^{R}Aerosil.

6. The process as claimed in claim 1, wherein the ion exchanger treatment is followed by precipitation of fibrinogen from the solution by addition of glycine in a concentration of 0.5 to 3 mol/l, preferably 2.7 mol/l, and the vWF is precipitated from the supernatant with an NaCl concentration of 2-15% (w/v), preferably 6% (w/v).

7. The process as claimed in claim 1, wherein the pasteurized and purified vWF is sterilized by filtration and lyophilized with the addition of glycine (2% w/v), albumin (0.5%) in citrated (0.02 mol/l) NaCl (0.06 mol/l).

## Revendications

1. Procédé de préparation d'un concentré de facteur von Willebrand (FvW) pasteurisé, caractérisé en ce qu'on traite avec un échangeur d'anions une solution qui contient le FvW sous forme de complexe avec F VIII:C dans un tampon pH 5,5-6,5 à base de calcium et d'acide aminé et avec une concentration d'hydrates de carbone de 5 à 30 % en poids, le F VIII:C se fixant sur l'échangeur, et en ce qu'on obtient le concentré de FvW à partir de la solution.

2. Procédé selon la revendication 1, caractérisé en ce qu'on utilise, comme solution renfermant le FvW a l'état de complexe avec le F VIII:C, du plasma ou une fraction provenant du plasma, de préférence un cryoprécipité ou une fraction de Cohn I, ou bien un surnageant ou extrait d'une culture cellulaire.

3. Procédé selon la revendication 1, caractérisé en ce qu'on pasteurise la solution renfermant le FvW.

4. Procédé selon la revendication 1, caractérisé en ce qu'on pasteurise la solution du FvW, on la protège, pendant la pasteurisation, contre une inactivation thermique par addition de saccharose, de préférence à une concentration de 10 à 60 % en poids/vol., de glycine, de préférence à une concentration de 0,5 à 3 moles/l, et d'un sel de calcium, de préférence à une concentration de 1 à 20 mmoles/l, et on empêche en même temps, grâce à ces additions, la précipitation de protéines sensibles aux acides, surtout du fibrinogène et de la fibronectine.

5. Procédé selon une quelconque des revendications 1 à 4, caractérisé en ce qu'on dissout les précipités de FvW, tels qu'ils se présentent avant ou après la pasteurisation, et on les traite avec Aerosil^{(R)}.

6. Procédé selon la revendication 1, caractérisé en ce que, après le traitement par échangeur d'ions, on fait précipiter le fibrinogène à partir de la solution par addition de glycine à une concentration de 0,5 à 3 moles/l, de préférence 2,7 moles/l, et on fait précipiter le FvW à partir du surnageant, avec une concentration de NaCl de 2 à 15 % (poids/vol.), de préférence 6 % (poids/vol.).

7. Procédé selon la revendication 1, caractérisé en ce que le FvW pasteurisé et purifié est soumis à une filtration stérile et à une lyophilisation, par addition de glycine (2 % en poids/vol.) et d'albumine (0,5 %) dans un tampon citrate (0,02 mole/l)-NaCl (0,06 mole/l).
